# EUROPÄISCHE PATENTSCHRIFT

(11) **EP 1 272 270 B1**
(45) Veröffentlichungstag und Bekanntmachung des Hinweises auf die Patenterteilung: **21.01.2009**
(21) Anmeldenummer: 01936136.9
(22) Anmeldetag: 23.03.2001
(51) Int. Cl.: B01J 23/36

(54) **VERFAHREN ZUR HERSTELLUNG VON 1,4-Butandiol AN RHENIUMHALTIGEN AKTIVKOHLE-TRÄGERKATALYSATOREN**
METHOD FOR THE PRODUCTION OF 1,4-butanediol ON RHENIUM-CONTAINING ACTIVATED CHARCOAL SUPPORTED CATALYSTS
PROCEDE POUR PRODUIRE de 1,4-butanediol SUR DES CATALYSEURS A SUPPORT CONSTITUE DE CHARBON ACTIF, CONTENANT DU RHENIUM

(30) Priorität: 24.03.2000 DE 10014646
(43) Veröffentlichungstag der Anmeldung: 08.01.2003
(73) Patentinhaber: BASF SE, 67056 Ludwigshafen (DE)
(72) Erfinder: FISCHER, Rolf-Hartmuth, 69121 Heidelberg (DE); PINKOS, Rolf, 67089 Bad Dürkheim (DE); SCHUNK, Stephan, Andreas, 69115 Heidelberg (DE); WULFF-DÖRING, Joachim, 67227 Frankenthal (DE)
(74) Vertreter: Isenbruck, Günter
(86) Internationale Anmeldenummer: PCT/EP2001/003374
(87) Internationale Veröffentlichungsnummer: WO 2001/070657

(56) Entgegenhaltungen:
- EP-A- 0 319 116
- EP-A- 0 373 938
- EP-A- 0 589 168
- EP-A- 0 848 991
- EP-A- 0 881 203
- US-A- 6 008 384

## Beschreibung

Die Erfindung betrifft ein Verfahren zur Herstellung von 1,4-Butandiol durch Hydrierung von Carbonylgruppen enthaltenden Verbindungen an Re-haltigen, oxidativ vorbehandelten Aktivkohle-Trägerkatalysatoren unter Vermeidung der Bildung von Ethern.

Bei der industriellen Herstellung von Alkoholen geht man häufig von Carbonylgruppen enthaltenden Edukten wie Aldehyden, Ketonen, Carbonsäuren, Cabonsäureanhydriden und Estern aus, die mit Wasserstoff hydriert werden.

In der jüngeren Vergangenheit wurden dabei unter Verwendung von oxidativ vorbehandelten Aktivkohleträgern besonders aktive Katalysatoren gefunden. In EP-A-0 848 991 ist ein Palladium, Silber, Rhenium und Eisen enthaltender Katalysator beschrieben, der z.B. Maleinsäure oder deren Ester zu Butandiol hydrieren kann. Bei der Hydrierung von Maleinsäure bei 100 bis 162°C wird eine Selektivität zu Butandiol von 89,5% erreicht. Der Hydriererfolg wird dadurch geschmälert, daß als Nebenprodukt als Ether 5,6% Tetrahydrofuran (THF) entsteht. Daneben entsteht noch als weiteres Nebenprodukt n-Butanol mit 4%.

In US 5,698,749 sind Katalysatoren beschrieben, die ein Element der Gruppe VIII und mindestens noch Rhenium, Wolfram oder Molybdän auf einem oxidativ vorbehandelten Kohleträger enthalten. Insbesondere werden Pd/Re/C-bzw. Pd/Re/Ag/C- Katalysatoren beschrieben. Mit diesen Katalysatoren entsteht bei der Hydrierung von wäßriger Maleinsäure wiederum neben Butandiol THF. Dabei wird Butandiol mit bis zu 92,8% Selektivität erhalten. THF entsteht jedoch immer noch zu 1,6%, das weitere Nebenprodukt n-Butanol zu 4,6%. Die Tendenz der Hydriermetalle Rhenium bzw. Platin, bei der Hydrierung von Maleinsäure-Derivaten THF und damit Ether zu bilden, ist bekannt (siehe z.B. A.F. Timofeev et al., Prikl. Khim. (Leningrad) 1981, 54 (2), 335-8, Chemical Abstracts 95: 80602 X). Der gleiche Effekt wird auch in GB-A-1 551 741 unter Verwendung von geträgerten Pd/Re-, Pt/Re- oder Pt/Pd/Re- Katalysatoren beschrieben.

H.S. Broadbent et al. beschreiben in J. Org. Chem. 24, 1847-1854 (1959) die Bernsteinsäurehydrierung an nicht geträgertem metallischem Re, bei der erhebliche Mengen an THF gebildet werden.

EP 0 881 203 A1 offenbart ein Verfahren zur Herstellung von 1,4-Butandiol umfassend die katalytische Hydrierung von hydrierbaren Vorläuferverbindungen in Kontakt mit einem Wasserstoff enthaltenden Gas und einem Hydrierkatalysator, enthaltend wenigstens ein Edelmetall der Gruppe VIII des Periodensystems der Elemente und wenigstens eines ausgewählt aus Rhenium, Wolfram und Molybdän auf einem Kohlenstoffträger, wobei der Träger zuvor mit einem Oxidationsmittel ausgewählt aus Salpetersäure, Wasserstoffperoxid, Natriumhypochlorit, Ammoniumpersulfat und Perchlorsäure behandelt worden ist.

EP 0 373 938 A2 offenbart einen Formaldehyd-resistenten Rhenium-Katalysator, welcher in der Hydrierung von Cabonylverbindungen, Acetalen und Estern zu Alkoholen eingesetzt werden kann, falls das Reaktionsmedium Formaldehyd als Verunreinigung enthält.

US 4,048,110 offenbart einen Rheniumkatalysator, der durch Adsorption einer Rhenium enthaltenden Verbindung auf einem Trägermaterial und anschließende thermische Zersetzung der Verbindung in Gegenwart von Wasserstoff erhalten wird. Solche Katalysatoren können gemäß US 4,048,110 zur selektiven Herstellung on alpha,beta-ungesättigten Alkoholen aus alpha,beta-ungesättigten Aldehyden in der Gasphase verwendet werden. Die Vermeidung von Ethern als Nebenprodukt ist bei technischen Hydrierungsprozessen jedoch wünschenswert, da deren Bildung die Wirtschaftlichkeit des Verfahrens vermindert. Ferner sind die Ether mitunter schwer vom gewünschten Produkt abzutrennen. Darüber hinaus verursachen die Ether erhebliche Entsorgungskosten, da sie, wie z.B. THF, bereits in kleinen Mengen nicht mehr in eine Kläranlage eingebracht werden dürfen, weil sie nur schwer biologisch abbaubar sind.

Aufgabe der vorliegenden Erfindung ist die Bereitstellung von Rhenium-Katalysatoren, mit denen man mit hoher Gesamtselektivität Carbonylverbindungen zu 1,4-Butandiol hydrieren kann, vorzugsweise ohne Ether zu bilden.

Es wurde nun gefunden, daß man Carbonylverbindungen ausgewählt aus Bernsteinsäure, Maleinsäure, Fumarsäure, sowie die Ester und Anhydride dieser Säuren und gamma-Butyrolacton katalytisch zu 1,4-Butandiol ohne Etherbildung hydrieren kann, indem man einen Katalysator aus 0,01 bis 50 Gew.-% Rhenium und 0 bis 20 Gew.-%, jeweils bezogen auf das Gesamtgewicht des Katalysators, mindestens eines weiteren Metalls, ausgewählt aus Zn, Cu, Ag, Au, Ni, Fe, Cr, V, auf mit Salpetersäure oxidativ vorbehandelter Aktivkohle als Träger zur Hydrierung einsetzt. Dabei heißt ohne Etherbildung, daß die Etherbildung höchstens 0,5% Anteil an den Hydrierprodukten haben soll. Bevorzugt liegt der Etheranteil unter 0,2%, besonders bevorzugt unter 0,1 %.

Die Erfindung betrifft ein Verfahren zur Herstellung von 1,4-Butandiol durch katalytische Hydrierung von Carbonylverbindungen ausgewählt aus Bernsteinsäure, Maleinsäure, Fumarsäure, sowie die Ester und Anhydride dieser Säuren und gamma-Butyrolacton, bei dem man als Katalysator 0,01 bis 50 Gew.-% Rhenium und 0 bis 20 Gew.-%, jeweils bezogen auf das Gesamtgewicht des Katalysators, mindestens eines weiteren Metalls, ausgewählt aus Zn, Cu, Ag, Au, Ni, Fe, Cr, V, auf mit Salpetersäure oxidativ vorbehandelter Aktivkohle als Träger einsetzt, und den entsprechenden Katalysator und seine Verwendung bei der katalytischen Hydrierung von Carbonylverbindungen.

Die zusätzlichen Elemente können den Katalysator im wesentlichen bezüglich der Aktivität und Selektivität in bezug auf die Hydrogenolyseprodukte modifizieren. Sie sind jedoch nicht essentiell.

Der Anteil an Rhenium (als Metall gerechnet) beträgt vorzugsweise 0,1 bis 20 Gew.-%, besonders bevorzugt 1 bis 15 Gew.-%, bezogen auf das Gesamtgewicht des Katalysators.

Insbesondere wird ein Katalysator eingesetzt, der nur aus Rhenium auf oxidativ vorbehandelter Aktivkohle als Träger besteht.

Vorzugsweise ist der Katalysator als Festbett angeordnet.

Als Aktivkohlen kommen im allgemeinen die handelsüblichen Aktivkohlen in Frage. Bevorzugt werden solche eingesetzt, die wenig Chlor und Schwefel enthalten und deren Mikroporenanteil möglichst gering ist. Die oxidative Behandlung der Aktivkohlen erfolgt mit Salpetersäure. Die Behandlung der Aktivkohle mit dem Oxidationsmittel erfolgt vor der Aufbringung der Rheniumkomponente bzw. weiterer Katalysatorkomponenten. Geeignete Verfahren sind auch in US 5,698,749 und EP-A-0 848 991 beschrieben.

Als Rheniumkomponente wird Re₂O₇ verwendet.

Die Aufbringung der Aktivkomponenten, insbesondere von Re, kann durch Imprägnierung in einem oder mehreren Schritten mit einer wäßrigen oder alkoholischen Lösung der jeweiligen gelösten Salze, Imprägnierung mit einer Lösung von gelöstem oxidischen oder metallischen Kolloid der Aktivkomponenten, Gleichgewichtsadsorption in einem oder mehreren Schritten der in wäßriger oder alkoholischer Lösung gelösten Salze oder Gleichgewichtsadsorption von gelöstem oxidischen oder metallischen Kolloid an der vorbehandelten Aktivkohle vorgenommen werden. Bei diesem Verfahren können die Aktivkomponenten entweder gleichzeitig oder nacheinander auf die Aktivkohle aufgebracht werden. Zwischen den einzelnen Imprägnierungs- und Gleichgewichtsadsorptionschritten liegt jeweils ein Trocknungsschritt zur Entfernung des Lösungsmittels. Bevorzugt geschieht die Aufbringung der Aktivkomponenten durch Imprägnierung mit einer wäßrigen Salzlösung oder einem wäßrigen oxidischen Kolloid in einem Schritt.

Zur Entfernung des Lösungsmittels nach dem Imprägnierungs- und Gleichgewichtsadsorptionschritt erfolgt eine Trocknung des imprägnierten Katalysators. Die Trocknungstemperatur liegt dabei bei 30 - 350°C, bevorzugt 40 - 280°C, besonders bevorzugt 50-150°C.

Die Katalysatoren werden üblicherweise vor ihrem Einsatz aktiviert. Bevorzugt wird dazu Wasserstoff verwendet. Die Aktivierungstemperatur liegt dabei bei 100 - 500°C, bevorzugt 130 - 400°C, besonders bevorzugt 150 - 350°C.

Die Hydrierung wird bei 50 - 250°C, bevorzugt bei 60 - 220°C, besonders bevorzugt bei 70 - 190°C, ganz besonders bevorzugt bei 80 - 140°C durchgeführt. Dabei wird bei einem Reaktionsdruck zwischen 3 und 330 bar, bevorzugt 20 und 300 bar, besonders bevorzugt 30 und 300 bar hydriert. Dabei wird der Druckbereich bei der Hydrierung in der Flüssigphase im Festbett über 150 bar, im Festbett in der Gasphase 3 bis 100 bar und in der Suspension 10 bis 90 bar bevorzugt.

Die Edukte für die Hydrierung sind ausgewählt aus Bernsteinsäure, Maleinsäure, Fumarsäure, sowie die Ester und Anhydride dieser Säuren und gamma-Butyrolacton.

Das zugängliche Produkt ist 1,4-Butandiol.

Die zu hydrierenden Verbindungen können in Substanz oder in Lösung hydriert werden. Als Lösungsmittel bietet sich z.B. das Hydrierprodukt selbst an, oder es werden unter den Reaktionsbedingungen inerte Stoffe eingesetzt wie Alkohole, z.B. Methanol, Ethanol, Propanol oder Butanol. Ferner sind Ether wie THF oder Ethylenglycolether geeignet. Ein bevorzugtes Lösungsmittel ist Wasser, insbesondere bei der Hydrierung von Carbonsäuren. Die Hydrierung kann in der Gas- oder Flüssigphase und ein- oder mehrstufig ausgeführt werden. In der Flüssigphase ist sowohl die Suspensions- als auch die Festbettfahrweise möglich. Bei exothermen Reaktionen kann die Wärme durch außenliegende Kühlmittel abgeführt werden (z.B. Röhrenreaktor). Ferner ist Siedekühlung im Reaktor möglich, vor allem, wenn ohne Produktrückführung hydriert wird. Bei Produktrückführung bietet sich ein Kühler im Rückführstrom an.

Vorzugsweise wird die Hydrierung in der Flüssigphase bei einem Druck im Bereich von 150 bis 300 bar und einer Temperatur im Bereich von 80 bis 140°C durchgeführt. Dabei wird der Katalysator insbesondere in Form eines Festbettes eingesetzt.

Das im erfindungsgemäßen Verfahren erhaltene 1,4-Butandiol wird z.B. als Lösemittel und Zwischenprodukt eingesetzt. Butandiol findet als Diolkomponente in Polyestern Verwendung.

Das erfindungsgemäße Verfahren wird anhand der nachstehenden Beispiele näher erläutert. Die angegebenen Gehalte der einzelnen Komponenten in den Hydrierausgängen sind gaschromatographisch ermittelt worden. Sie sind, wenn nicht anders angegeben, lösungsmittelfrei gerechnet.

### Beispiele

### Beispiel 1:

20 g Aktivkohle (1-3 mm Splitt) wurden mit 65% HNO₃ oxidiert und bei 120°C getrocknet. Auf 18 g der so vorbehandelten Kohle wurden 5 g Re₂O₇ als wäßrige Lösung aufgetränkt und bei 120°C getrocknet. Der so gewonnene Katalysator wurde anschließend mit Wasserstoffstrom 40 h bei 270°C und Umgebungsdruck aktiviert. Vom aktivierten Katalysator wurden anschließend 25 ml in einen 25 ml fassenden Reaktor eingefüllt. Die Hydrierung erfolgte in Rieselfahrweise ohne Produktrückführung, der Zulauf betrug ca. 20 g/h. Der Reaktionsdruck lag bei 220 bar, es wurden ca. 100 NL Wasserstoff/h eingeleitet. Begonnen wurde mit einer 5%igen Maleinsäurelösung in Wasser bei einer Temperatur von 174°C.

Im Austrag fanden sich nach 27 h Versuchszeit ca. 96,5%.Butandiol und 3,5% n-Butanol. Nach Absenkung der Temperatur auf 166°C fanden sich im Hydrieraustrag nach insgesamt 45 Versuchsstunden ca. 97,1% Butandiol, 0,9% gamma-Butyrolacton und 2% n-Butanol. Nach Erhöhung der Maleinsäurekonzentration im Zulauf auf 20% wurden bei 166°C und einer Gesamtlaufzeit von 55 h 95,7% Butandiol, 2,5% gamma-Butyrolacton und 1,5% n-Butanol gefunden. Während der gesamten Versuchszeit wurde im Hydrierausgang kein THF nachgewiesen.

### Beispiel 2 (nicht erfindungsgemäß):

Analog Beispiel 1 wurden 20 g Aktivkohle (Epibon® Spezial, von Lurgi) mit HNO₃ vorbehandelt und mit 5 g Re₂O₇ sowie 15 g Platinnitratlösung (= 2,5% PtO₂) getränkt und getrocknet. Danach wurde wie in Beispiel 1 weiter vorgegangen mit der Ausnahme, daß nach 24 h Versuchszeit auf 30%ige Maleinsäurelösung umgestellt wurde. Nach insgesamt 78 h Versuchszeit wurden bei ca. 100°C Reaktionstemperatur im Austrag ca. 97,3% Butandiol und 2,5% n-Butanol, aber kein THF gefunden.

## Patentansprüche

1. Verfahren zur Herstellung von 1,4-Butandiol durch katalytische Hydrierung von Carbonylverbindungen ausgewählt aus, Maleinsäure, Fumarsäure, Bernsteinsäure oder Estern oder Anhydriden davon, oder gamma-Butyrolacton, **dadurch gekennzeichnet, daß** der Katalysator aus 0,01 bis 50 Gew.% Rhenium und 0 bis 20 Gew.%, jeweils bezogen auf das Gesamtgewicht des Katalysators, mindestens eines weiteren Metalls, ausgewählt aus Zn, Cu, Ag, Au, Ni, Fe, Cr, V, auf oxidativ vorbehandelter Aktivkohle als Träger besteht.

2. Verfahren nach Anspruch 1, **dadurch gekennzeichnet, daß** der Katalysator aus 0,1 bis 20 Gew.-% Rhenium, bezogen auf das Gesamtgewicht des Katalysators, auf oxidativ vorbehandelter Aktivkohle als Träger besteht.

3. Verfahren nach Anspruch 1 oder 2, **dadurch gekennzeichnet, daß** der Katalysator als Festbett angeordnet ist.

4. Verfahren nach einem der Ansprüche 1 bis 3, **dadurch gekennzeichnet, daß** die Hydrierung in der Flüssigphase bei einem Druck im Bereich von 150 bis 300 bar und bei einer Temperatur im Bereich von 50 bis 250°C durchgeführt wird.

5. Katalysator, bestehend aus 0,01 bis 50 Gew.-% Rhenium und 0 bis 20 Gew.-%, jeweils bezogen auf das Gesamtgewicht des Katalysators, mindestens eines weiteren Metalls, ausgewählt aus Zn, Cu, Ag, Au, Ni, Fe, Cr, V, auf oxidativ vorbehandelter Aktivkohle als Träger, wobei Rhenium als Re₂ O₇ auf den Träger aufgebracht wird.

6. Katalysator nach Anspruch 5, **dadurch gekennzeichnet, dass** der Katalysator aus 0,1 bis 20 Gew-% Rhenium, bezogen auf das Gesamtgewicht des Katalysators, auf oxidativ vorbehandelter Aktivkohle als Träger besteht.

7. Verwendung eines Katalysators gemäß Anspruch 5 oder 6 bei der katalytischen Hydrierung von Carbonylverbindungen.

## Claims

1. A process for the preparation of 1,4-butanediol by catalytic hydrogenation of carbonyl compounds chosen from maleic acid, fumaric acid, succinic acid or esters or anhydrides thereof, or gamma-butyrolactone, wherein the catalyst consists of 0.01 to 50% by weight of rhenium and 0 to 20% by weight, in each case based on the total weight of the catalyst, of at least one further metal chosen from Zn, Cu, Ag, Au, Ni, Fe, Cr, V on activated carbon oxidatively pretreated with nitric acid as support.

2. The process according to claim 1, wherein the catalyst consists of 0.1 to 20% by weight of rhenium, based on the total weight of the catalyst, on oxidatively pretreated activated carbon as support.

3. The process according to claim 1 or 2, wherein the catalyst is arranged as a fixed bed.

4. The process according to any of claims 1 to 3, wherein the hydrogenation is carried out in the liquid phase at a pressure in the range from 150 to 300 bar and at a temperature in the range from 50 to 250°C.

5. A catalyst consisting of 0.01 to 50% by weight of rhenium and 0 to 20% by weight, in each case based on the total weight of the catalyst, of at least one further metal chosen from Zn, Cu, Ag, Au, Ni, Fe, Cr, V on activated carbon oxidatively pretreated with nitric acid as support, rhenium being applied to the support as Re₂O₇.

6. The catalyst according to claim 5, wherein the catalyst consists of 0.1 to 20% by weight of rhenium, based on the total weight of the catalyst, on oxidatively pretreated activated carbon as support.

7. The use of a catalyst according to claim 5 or 6 in the catalytic hydrogenation of carbonyl compounds.

## Revendications

1. Procédé de préparation de 1,4-butanediol par hydrogénation catalytique de composés carbonylés choisis parmi l'acide maléique, l'acide fumarique, l'acide succinique ou leurs esters ou leurs anhydrides ou le gamma-butyrolactone, **caractérisé en ce que** le catalyseur est constitué, par rapport à son poids total, d'au moins 0,01 à 50 % en poids de rhénium ainsi que de 0 à 20 % en poids d'un moins un autre métal choisi parmi Zn, Cu, Ag, Au, Ni, Fe, Cr, V, sur un support en charbon actif ayant subi un prétraitement d'oxydation à l'acide nitrique.

2. Procédé selon la revendication 1, **caractérisé en ce que** le catalyseur est constitué, par rapport à son poids total, de 0,1 à 20 % en poids de rhénium sur un support en charbon actif ayant subi un prétraitement d'oxydation.

3. Procédé selon les revendications 1 ou 2, **caractérisé en ce que** le catalyseur disposé sous forme de lit fixe.

4. Procédé selon l'une des revendications 1 à 3, **caractérisé en ce que** l'hydrogénation est réalisée en phase liquide, la pression étant comprise entre 150 et 300 bar et la température étant comprise entre 50 et 250°C.

5. Catalyseur constitué, par rapport à son poids total, de 0,01 à 50 % en poids de rhénium et de 0 à 20 % en poids d'au moins un autre métal choisi parmi Zn, Cu, Ag, Au, Ni, Ce, Cr, V sur un support en charbon actif ayant subi un prétraitement d'oxydation à l'acide nitrique, le rhénium étant appliqué sur le support sous forme de Re₂O₇.

6. Catalyseur selon la revendication 5, **caractérisé en ce que** le catalyseur est constitué, par rapport à son poids total, de 0,1 à 20 % en poids de rhénium sur un support en charbon actif ayant subi un prétraitement d'oxydation.

7. Utilisation d'un catalyseur selon les revendications 5 ou 6 lors de l'hydrogénation catalytique de composés carbonylés.
